# EUROPEAN PATENT SPECIFICATION

(11) **EP 0 904 089 B1**
(45) Date of publication and mention of the grant of the patent: **11.09.2002**
(21) Application number: 97918822.4
(22) Date of filing: 25.04.1997
(51) Int. Cl.: A61K 33/04, A61K 33/24, A61K 31/70, A61K 31/765, A61K 31/78, A61K 31/715, A61L 24/00

(54) **STERILIZATION OF FEMALE MAMMALS**
STERILISATION WEIBLICHER SÄUGETIEREN
STERILISATION DE MAMMIFERES FEMELLES

(30) Priority: 31.05.1996 US 656394; 11.12.1996 US 763724
(43) Date of publication of application: 31.03.1999
(73) Proprietor: MICRO THERAPEUTICS, INC., San Clemente, CA 92673 (US)
(72) Inventor: WALLACE, George, Coto De Caza, CA 92679 (US); EVANS, Scott, Santa Ana, CA 92705 (US); GREFF, Richard, J., St. Pete Beach, FL 33706 (US)
(74) Representative: Des Termes, Monique
(86) International application number: US9707054
(87) International publication number: WO97045130

(56) References cited:
- EP-A- 0 050 457
- WO-A-85/00969
- WO-A-97/04657
- SETHI N. ET AL: "Histological changes in the vas deferens of rats after injection of a new male antifertility agent "SMA" and its reversiblity" CONTRACEPTION, vol. 41, no. 3, 1990, pages 333-339, XP002038212
- ZHAO SHENG-CAI: "Vas deferens occlusion by percutaneous injection of polyurethane elastomer plugs: Clinical experience and reversiblity" CONTRACEPTION, vol. 41, 1990, pages 453-459, XP002038213
- CHVAPIL M. ET AL: "Occlusion of the vas deferens in dogs with a biocompatible hydrogel solution" J. REPRODUCTIVE MEDICINE, vol. 35, no. 9, 1990, pages 905-910, XP002038214
- MANDAI S. ET AL: "Direct thrombosis of aneurysms with cellulose acetate polymer" J. NEUROSURG., vol. 77, 1992, pages 497-500, XP002038215

## Description

### BACKGROUND OF THE INVENTION

### Field of the Invention

This invention relates to means for sterilizing female mammals generally and female humans in particular.

A composition comprising a biocompatible polymer, a biocompatible solvent and a contrast agent is delivered to the fallopian tube of the female mammal. The biocompatible polymer is selected to be soluble in the biocompatible solvent, but insoluble in the aqueous fluid of the fallopian tube. The biocompatible solvent is miscible or soluble in this aqueous fluid and, upon contact with this fluid, the biocompatible solvent quickly diffuses away whereupon the biocompatible polymer precipitates to form an occlusion in the fallopian tube which blocks the passage of eggs from the ovary

A significant advantage is that the sterilization can be reversed merely by dissolving the biocompatible polymer forming the occlusion with the biocompatible solvent.

### References

The following publications are cited in this application as superscript numbers:
¹ Rakel, *Textbook of Family Practice*, 5th Ed., pp. 726, W.B. Saunders, Philadelphia, PA (1995)
² Cunningham, *Williams Obstetrics,* Chapter 62, *Surgical Contraception,* pp. 1353-1359, Appleton & Lange, Norwalk, CT (1993)
³ Dewitt, *"Surgery of the Male Genital Tract*", in *Family Medicine Principles and Practice*, 4th Edition, Taylor, Editor, pp. 778-780 (1994)
⁴ Stoy, et al., *U.S. Patent No. 4,631,188*, for "Injectable Physiologically-Acceptable Polymeric Composition", issued December 23, 1986
⁵ Rabinowitz, et al., *U.S. Patent No. 3,527,224*, for "Method of Surgically Bonding Tissue Together", issued September 8, 1970.
⁶ Hawkins, et al., *U.S. Patent No. 3,591,676*, for "Surgical Adhesive Compositions", issued July 6, 1971.
⁷ Kinugasa, et al., "Direct Thrombosis of Aneurysms with Cellulose Acetate Polymer", *J. Neurosurg*., 77:501-507 (1992).
⁸ Greff, et al., *U.S. Parent Application Serial No. 08*/*507,863* for "Novel Compositions for Use in Embolizing Blood Vessels", filed July 27, 1995.
⁹ Greff, et al., *U.S. Patent Application Serial No. 08*/*508,248* for "Cellulose Diacetate Compositions for Use in Embolizing Blood Vessels", filed July 27, 1995.
¹⁰ Kinugasa, et al., "Early Treatment of Subarachnoid Hemorrhage After Preventing Rerupture of an Aneurysm", *J. Neurosurg*., 83:34-41 (1995).
¹¹ Kinugasa, et al., "Prophylactic Thrombosis to Prevent New Bleeding and to Delay Aneurysm Surgery", *Neurosurg*., 36:661 (1995).
¹² Taki, et al., "Selection and Combination of Various Endovascular Techniques in the Treatment of Giant Aneurysms", *J. Neurosurg*., 77:37-42 (1992).
¹³ Park, et al., "New Polymers for Therapeutic Embolization", Poster #47, Meeting of the Radiological Society of North America (1993)

### State of the Art

Normal surgical contraceptive methods include both vasectomy (males) and tubal ligation (females).^{1,2,3} For females, tubal ligation has been recognized as one of the safest, simplest, and most effective forms of sterilization with mortality rates as low as 3 per 100,000.² As normally practiced, tubal ligation involves a skin incision and subsequent inactivation of the fallopian tubes. Procedures recognized in the art of tubal ligation include the Irving procedure, the Pomeroy procedure, the Madlener procedure, the Parkland procedure and the Kroener fimbriectomy procedure each of which is discussed and illustrated by Cunningham².

Notwithstanding its safety and efficacy, tubal ligation has not gained wide acceptance as a form of female contraception partially because it is a surgical procedure requiring a sterile environment and has possible complications including hemorrhage, infection, anesthetic complications, scarring, formation of adhesions, and the like.²

Another drawback of tubal ligation is a lack of reversibility. While some female mammals have successfully reproduced after having surgical sterilization, the success rate is not high enough to make the procedure more appealing as a form of female contraception and the procedure is generally construed as irreversible.¹

In view of the above drawbacks, attempts have been made to develop a non-surgical and reversible method of female sterilization. One such attempt involves the injection of a polymeric hydrogel solution in a suitable solvent such as DMSO into the fallopian tubes.⁴ Upon injection into the fallopian tubes, the polymeric composition slowly coagalugates into a spongy polymer structure which takes up water to from a hydrogel composition.

However, the procedures described in Stoy⁴ are hampered by the fact that the injection procedure is difficult to monitor and it is also difficult to determine whether the procedure, when completed, has been successful. Moreover, Stoy's hydrogels are reported as causing osmotic shock to neighboring tissue and the coagulation process is rather slow resulting in a sponganeous polymer forming *in situ.* This slow coagulation process could lend itself to migration of at least some of the polymer from the intended site of application prior to formation of the sponganeous polymer with the concomitant possibility that the sterilization process will be unsuccessful.

In view of the above drawbacks, a need continues to exist in the art for an easy, reliable, and dependable method of sterilizing female mammals.

Sethi N. et al., Contraception, vol. 41, n°3, 1990, p. 333-339 disclose the use of a polymer, styrene maleic anhydride (SMA) dissolved in dimethylsulphoxide for male sterilization.

EP-A-050 457 discloses the use of a group of compounds which may be added to methylcyanoacrylate and other cyanoacrylate esters in order to provide radiopaque compositions useful in medical and industrial applications.

This invention is directed to the discovery that the efficacy of female sterilization by placement of a polymer composition dissolved in a solvent can be enhanced by further delivery of a contrast agent as described below into the fallopian tubes. The contrast agent permits monitoring of the injection while it is taking place to ensure that the sterilization procedure is being carried out properly. The methods described herein can also be reversed quickly and reliably to restore the female's reproductive capacity. Moreover, the use of a water insoluble contrast agent in the sterilization procedure has the added advantage of providing a facile means to locate the polymeric blockage in the fallopian tubes potentially years after the polymer has been placed there.

### SUMMARY OF THE INVENTION

This invention is based on the discovery that unexpected and surprising results are achieved when female mammals are sterilized with a composition comprising a biocompatible polymer, a biocompatible solvent, an inorganic contrast agent selected from the group consisting of tantalum, tantalum oxide, barium sulfate, gold, tungsten and platinum. In particular, deficiencies associated with each of the prior art procedures are either reduced or eliminated by the invention. Such deficiencies include, for example, problems associated with a skin incision and problems associated with readily reversing the sterilization process.

Accordingly, this invention is directed to the use of a composition comprising a biocompatible polymer, an inorganic contrast agent selected from the group consisting of tantalum, tantalum oxide, barium sulfate, gold, tungsten and platinum, and a biocompatible solvent, for the manufacture of a medicament for sterilizing a female mammal by delivering said composition to the fallopian tube of the female mammal under conditions such that a polymer precipitate forms in situ in the fallopian tube thereby sterilizing the female mammal.

In the composition, the biocompatible polymer is preferably an ethylene vinyl alcohol copolymer or a cellulose acetate polymer. The biocompatible solvent is preferably dimethylsulfoxide.

In another aspect, this invention is directed to the use of a composition comprising a biocompatible polymer, an inorganic contrast agent selected from the group consisting of tantalum, tantalum oxide, barium sulfate, gold, tungsten and platinum, and a first biocompatible solvent, for the manufacture of a medicament for reversibly sterilizing a female mammal by delivering said composition to the fallopian tube of the female mammal under conditions such that a polymer precipitate forms in situ in the fallopian tube thereby sterilizing the female mammal, and said sterilization being reversed by contacting said polymer precipitate formed in the fallopian tube with a second biocompatible solvent under conditions such that said polymer dissolves in said second biocompatible solvent thereby reversing said sterilization of the female mammal.

According to the invention, the contrast agent is a water insoluble contrast agent which, upon precipitation of the polymer *in situ*, will form part of the precipitate. Upon reversing the sterilization process, the contrast agent retained in the polymeric composition is employed to identify the location of the polymeric blockage in the fallopian tube.

In one embodiment, the first biocompatible solvent and the second biocompatible solvent are the same.

In the composition, the biocompatible polymer is preferably an ethylene vinyl alcohol copolymer or a cellulose acetate polymer. The first and second biocompatible solvents are preferably dimethylsulfoxide.

### DETAILED DESCRIPTION OF THE INVENTION

This invention is directed to the use of a composition for the manufacture of a medicament for use in a method for sterilizing female mammals, which method comprises delivering a composition comprising a biocompatible polymer, a biocompatible solvent, and a contrast agent to the fallopian tube of the female mammal.

Prior to discussing this invention in further detail, the following terms will first be defined:

The term "sterilizing" refers to a process for making a person or an animal unable to produce offspring. In the context of this invention, sterilization is carried out by delivering a material into the fallopian tube of the female mammal. The material then fills or plugs the fallopian tube so that eggs cease to pass therethrough.

The term "biocompatible polymer" refers to polymers which, in the amounts employed, are non-toxic, chemically inert, and substantially non-immunogenic when used internally in the female mammal and which are substantially insoluble in the fluid of the fallopian tube. The chemically inert polymers do not appreciably absorb water upon contact with the fluid of the fallopian tubes and typically will have an equilibrium water content of less than about 25% water and preferably less than about 15% water. Suitable biocompatible polymers include, by way of example, cellulose acetates^{7,10,11} (including cellulose diacetate⁹), ethylene vinyl alcohol copolymers^{8,12}, polyalkyl(C₁-C₆) acrylates, polyalkyl alkacrylates wherein the alkyl and the alk groups contain no more than 6 carbon atoms, polyacrylonitrile and the like. Further examples of biocompatible polymers are provided by Park, et al.¹³ Preferably, the biocompatible polymer is also non-inflammatory when employed *in vivo*.

The particular biocompatible polymer employed is not critical and is selected relative to the viscosity of the resulting polymer solution, the solubility of the biocompatible polymer in the biocompatible solvent, and the like. Such factors are well within the skill of the artisan.

Preferred biocompatible polymers include cellulose diacetate and ethylene vinyl alcohol copolymer. Cellulose diacetate polymers are either commercially available or can be prepared by art-recognized procedures. In a preferred embodiment, the number average molecular weight, as determined by gel permeation chromatography, of the cellulose diacetate composition is from about 25,000 to about 100,000; more preferably from about 50,000 to about 75,000; and still more preferably from about 58,000 to 64,000. The weight average molecular weight of the cellulose diacetate composition, as determined by gel permeation chromatography, is preferably from about 50,000 to 200,000 and more preferably from about 100,000 to about 180,000. As is apparent to one skilled in the art, with all other factors being equal, cellulose diacetate polymers having a lower molecular weight will impart a lower viscosity to the composition as compared to higher molecular weight polymers. Accordingly, adjustment of the viscosity of the composition can be readily achieved by mere adjustment of the molecular weight of the polymer composition.

Ethylene vinyl alcohol copolymers comprise residues of both ethylene and vinyl alcohol monomers. Small amounts (e.g., less than 5 mole percent) of additional monomers can be included in the polymer structure or grafted thereon provided such additional monomers do not alter the embolizing properties of the composition. Such additional monomers include, by way of example only, maleic anhydride, styrene, propylene, acrylic acid, vinyl acetate, and the like.

Ethylene vinyl alcohol copolymers are either commercially available or can be prepared by art-recognized procedures. Preferably, the ethylene vinyl alcohol copolymer composition is selected such that a solution of 6 weight percent of the ethylene vinyl alcohol copolymer, 35 weight percent of a tantalum contrast agent in DMSO has a viscosity equal to or less than 60 centipoise at 20°C. As is apparent to one skilled in the art, with all other factors being equal, copolymers having a lower molecular weight will impart a lower viscosity to the composition as compared to higher molecular weight copolymers. Accordingly, adjustment of the viscosity of the composition as necessary for catheter or needle delivery can be readily achieved by mere adjustment of the molecular weight of the copolymer composition.

As is also apparent, the ratio of ethylene to vinyl alcohol in the copolymer affects the overall hydrophobicity/hydrophilicity of the composition which, in turn, affects the relative solubility of the composition in the biocompatible solvent as well as the rate of precipitation of the copolymer in an aqueous solution (e.g:, blood). In a particularly preferred embodiment, the copolymers employed herein comprise a mole percent of ethylene of from about 25 to about 60 and a mole percent of vinyl alcohol of from about 40 to about 75. More preferably, these copolymers comprise from about 40 to about 60 mole percent of vinyl alcohol and from about 60 to 40 mole percent of ethylene. These compositions provide for requisite precipitation rates suitable for use in sterilizing female mammals.

The term "contrast agent" refers to a biocompatible (non-toxic) radiopaque material capable of being monitored during injection into a mammalian subject by, for example, radiography. The contrast agent is water insoluble. Water insoluble contrast agents include tantalum, tantalum oxide, and barium sulfate, each of which is commercially available in the proper form for *in vivo* use including a particle size of about 10 µm or less. Other water insoluble contrast agents include gold, tungsten, and platinum powders.

Preferably, the contrast agent has a water solubility of less than 0.01 mg/ml at 20°C.

The term "biocompatible solvent" refers to an organic material liquid at least at body temperature of the female mammal in which the biocompatible polymer is soluble and, in the amounts used, is substantially non-toxic. Suitable biocompatible solvents include, by way of example, dimethylsulfoxide, analogues/homologues of dimethylsulfoxide, ethanol, acetone, and the like. Aqueous mixtures with the biocompatible solvent can also be employed provided that the amount of water employed is sufficiently small that the dissolved polymer precipitates upon contact with the fallopian tube fluid, e.g., less than about 5%. Preferably, the biocompatible solvent is dimethylsulfoxide (DMSO).

### Compositions

The polymer composition employed in this invention are prepared by conventional methods whereby each of the components is added and the resulting composition mixed together until the overall composition is substantially homogeneous.

For example, polymer compositions can be prepared by adding sufficient amounts of the biocompatible polymer to the biocompatible solvent to achieve the effective concentration for the polymer composition. Preferably, the polymer composition will comprise from about 2.5 to about 8.0 weight percent of the biocompatible polymer composition based on the total weight of the polymer composition, including contrast agent and solvent, and more preferably from about 4 to about 5.2 weight percent. If necessary, gentle heating and stirring can be used to effect dissolution of the biocompatible polymer into the biocompatible solvent, e.g., 12 hours at 50°C.

Sufficient amounts of the contrast agent are then added to the solution to achieve the effective concentration for the complete polymer composition. Preferably, the polymer composition will comprise from about 10 to about 40 weight percent of the contrast agent and more preferably from about 20 to about 40 weight percent and even more preferably 35 weight percent based on the total weight of the composition including the biocompatible polymer and bioccmpatible solvent. When the contrast agent is not soluble in the biocompatible solvent, stirring is employed to effect homogeneity of the resulting suspension. In order to enhance formation of the suspension, the particle size of the contrast agent is preferably maintained at about 10 µm or less and more preferably at from about 1 to about 5 µm (e.g., an average size of about 2 µm).

The particular order of addition of components to the biocompatible solvent is not critical and stirring of the resulting suspension is conducted as necessary to achieve homogeneity of the composition. Preferably, mixing/stirring of the composition is conducted under an anhydrous atmosphere at ambient pressure. The resulting composition may be heat sterilized and then stored preferably in sealed amber bottles or vials until needed.

In order to enhance formation of the suspension, the particle size of the contrast agent is preferably maintained at about 10 µm or less and more preferably at from about 1 to about 5 µm (e.g., an average size of about 2 µm). In one preferred embodiment, the particle size of a water insoluble contrast agent is prepared, for example, by fractionation. In such an embodiment, a water insoluble contrast agent such as tantalum having a particle size of less than about 20 micron is added to an organic liquid such as ethanol (absolute) preferably in a clean environment. Agitation of the resulting suspension followed by settling for approximately 40 seconds permits the larger particles to settle faster. Removal of the upper portion of the organic liquid followed by separation of the liquid from the particles results in a reduction of the particle size which is confirmed under a microscope. The process is optionally repeated until a desired particle size is reached.

The particular order of addition of components is not critical and stirring of the resulting suspension is conducted as necessary to achieve homogeneity of the composition. Preferably, mixing/stirring of the composition is conducted under an anhydrous atmosphere at ambient pressure. The resulting composition is sterilized and then stored preferably in sealed amber bottles or vials until needed.

### Methods

The compositions described above are then employed in methods for sterilizing female mammals. In these methods, the composition is introduced to the fallopian tube via conventional laproscopic, catheter, or needle technology.

Upon discharge of the composition into the fallopian tube, the biocompatible solvent dissipates into the fallopian tube fluid resulting in the precipitation of the biocompatible polymer. The precipitate forms in the fallopian tube which acts as a plug to stop the flow of eggs from the ovary to the uterus.

The particular amount of polymer composition employed is dictated by the diameter of the fallopian tube, the concentration of polymer in the composition, the rate of precipitation (solids formation) of the polymer, etc. Such factors are well within the skill of the artisan. For example, the rate of precipitation can be controlled by changing the overall hydrophobicity/hydrophilicity of the polymer with faster precipitation rates being achieved by a more hydrophobic polymer composition.

One particularly preferred method for delivering the composition to the fallopian tube is via a small diameter medical catheter. The particular catheter employed is not critical provided that polymeric catheter components are compatible with the polymeric composition (i.e., the catheter components will not readily degrade in the polymer composition and none of the components of the polymer compositions will readily degrade in the presence of the catheter components). In this regard, it is preferred to use polyethylene in the catheter components because of its inertness in the presence of the polymeric composition described herein. Other materials compatible with the composition can be readily determined by the skilled artisan and include, for example, other polyolefins, fluoropolymers (e.g., Teflon®), silicone, etc.

When delivered by catheter, the injection rate of the polymer composition dictates, in part, the form of the precipitate in the fallopian tube. Specifically, low injection rates of approximately 0.05 to 0.3 cc/minute will provide for a precipitate in the form of a kernel or nodule which is particularly beneficial because the precipitate forms primarily at the point of injection.

When introduced, the biocompatible solvent rapidly diffuses into the fluid present in the fallopian tube leaving a solid precipitate. The precipitate is a combination of the biocompatible polymer and the contrast agent. Without being limited to any theory, it is believed that initially, a soft gel to spongy solid precipitate forms upon contact with the fallopian tube fluid. This precipitate then restricts the migration of eggs from the ovary to the uterus thereby sterilizing the female mammal.

The methods described herein facilitate female sterilization because the presence of the contrast agent in the composition permits monitoring of the delivery of the biocompatible polymer while it is taking place. In this way, one can ensure that the biocompatible polymer is being delivered to the fallopian tubes as well as determine whether the size of the polymer precipitate thus-formed will be sufficient to block the passage of eggs.

In another aspect, the above-described sterilization procedure can be easily and reliably reversed. In such a case, the same procedures as sterilization are carried out except without the use of a biocompatible polymer and contrast agent. Specifically, a composition comprising a biocompatible solvent is delivered to the fallopian tube at or near the location of the polymer precipitate previously deposited therein. The biocompatible solvent acts to dissolve and flush out the polymer precipitate. The fallopian tube is thereby restored to its previous condition and eggs can again pass therethrough.

Without being limited to any theory, the methods described herein address the prior art problems recited above because the polymer precipitate formed in the fallopian tube can be dissolved and flush out with a biocompatible solvent after a period of time. Thus, the reproductive capacity of the female mammal may be restored safely and effectively. Additionally, if the polymer composition is delivered via conventional needle technology, the necessity of a skin incision can be avoided.

### Utility

The methods described herein are useful in sterilizing female mammals which, in turn, can be used to prevent/control reproduction. Accordingly, these methods find use in human and other mammalian subjects requiring sterilization.

The following examples are set forth to illustrate the claimed invention and are not to be construed as a limitation thereof.

### EXAMPLES

Unless otherwise stated, all temperatures are in degrees Celsius. Also, in these examples and elsewhere, the following abbreviations have the following meanings:
- cc =: cubic centimeter
- DMSO =: dimethylsulfoxide
- EVOH =: ethylene vinyl alcohol copolymer
- mm =: millimeter
- µm =: micron

In the following examples, Examples 1-2 illustrate the preparation of polymer compositions useful in the methods described herein which polymer compositions comprise cellulose acetate and EVOH. Examples 3 and 4 illustrate how such polymer compositions could be used in the methods of this invention.

### EXAMPLE 1

A cellulose diacetate polymer composition was prepared by dissolving cellulose acetate (39.7 weight percent acetyl content) into DMSO to provide for an 6.8 weight percent concentration of the polymer in DMSO. To this solution was added either tantalum (10 weight percent, available from Leico Industries, New York, New York, USA, 99.95% purity, less than 43 µm in size) as a water insoluble contrast agent or metrizamide (38.5 weight percent, available from Aldrich Chemical Company, Milwaukee, Wisconsin, USA) as a water soluble contrast agent.

In the tantalum composition, tantalum settling can result from prolonged standing. Sonification may help but thorough mixing prior to use is required.

In the Example above, tantalum powder can also be obtained from Aldrich Chemical Company, Milwaukee, Wisconsin, USA.

Preferably the composition comprises from about 25 to about 35 weight percent tantalum.

### EXAMPLE 2

An EVOH polymer composition was prepared by dissolving EVOH (44 mole percent ethylene) into DMSO to provide for an 6.8 weight percent concentration of the copolymer in DMSO. In order to facilitate dissolution, the system can be heated to 50°C overnight.

To this solution was added either tantalum (10 weight percent, available from Leico Industries, New York, New York, USA, 99.95% purity, less than 43 µm in size) as a water insoluble contrast agent or metrizamide (38.5 weight percent, available from Aldrich Chemical Company, Milwaukee, Wisconsin, USA) as a water soluble contrast agent.

In the tantalum composition, tantalum settling can result from prolonged standing. Sonification may help but thorough mixing prior to use is required.

Preferably the composition comprises from about 25 to about 35 weight percent tantalum.

### EXAMPLE 3

The purpose of this example is to illustrate how an *in vivo* application of the methods of this invention in the sterilization of a female mammal could be accomplished.

In this example, a 40 pound female dog is prepared for sterilization using a composition comprising 5.8 weight percent EVOH polymer (containing 48 weight percent ethylene), 20 weight percent tantalum in DMSO. This composition is loaded into a syringe having a long needle. The needle is then inserted into the lower abdominal area of the subject. The progress of the needle and the location of the fallopian tube can be monitored by conventional fluoroscopic techniques. Once the needle punctures the fallopian tube, the EVOH polymer composition (0.3 cc) is then delivered from the syringe to the fallopian tube. The delivery is easily visualized with fluoroscopy due to the presence of a contrast agent in the polymer composition. After delivery, the DMSO in the EVOH composition rapidly diffuses and the EVOH precipitates in the fallopian tube resulting in blockage of the tube. After about 5 minutes, the polymer is fully precipitated and the needle is removed from fallopian tube.

The same procedure is repeated with the other fallopian tube of the female subject.

### EXAMPLE 4

The purpose of this example is to illustrate how an *in vivo* application of the methods of this invention in reversing the sterilization of a female mammal could be accomplished.

In this example, the procedures of Example 3 are followed except that the polymer composition is replaced with the biocompatible solvent. Approximately 0.3 to 0.5 cc of DMSO is injected into the occluded fallopian tube over a period of 1 to 2 minutes to dissolve the previously deposited polymer precipitate which is removed by pulling back on the syringe. The procedure is repeated twice more. The dissolving and flushing of the precipitate is easily visualized with fluoroscopy due to the presence of a contrast agent in the polymer. After about 10 minutes, the polymer is fully dissolved and evacuated, and the syringe needle is removed from the fallopian tube.

### Example 5

The purpose of this example is to illustrate *ex vivo* reversibility of the process. Specifically, six (6) segments of coronary arteries were excised from fresh lamb hearts, obtained from a local meat store that day. The arteries (vessels) were approximately 6 cm in length and varied in diameter from about 1.5 to about 3.0 mm. Each segment was washed and then flushed with normal saline at room temperature.

The vessel segments were placed in a beaker filled with normal saline and a polymer composition comprising 7 weight percent cellulose acetate polymer (39% acetyl content) and 30 weight percent tantalum in DMSO was injected from a 3 cc syringe into each vessel through a 20 gage needle. Approximately 1 to 3 cm of each vessel was filled with the polymer composition and injection was over a 10 to 15 second period. Attempts to flush the vessel with normal saline showed no flow or total vessel occlusion.

After 15 minutes, a new 3 cc syringe and 20 gage needle filled with DMSO was introduced into the vessel, just proximal to the polymer plug. Gentle injection/aspiration of the DMSO over a 1 minute period yielded a noticeable dissolution of the polymer, with recanalization of the vessel within 2 to 3 minutes. Dissolution of the polymer plug took about 5 minutes. This result was repeated in all vessel segment samples.

It is understood that the same procedures set forth above can be employed with compositions employing liquid prepolymers. However, when so employed, the timing and injection rates will vary depending on the cure rate for the prepolymer. Such factors are within the skill of the artisan.

From the foregoing description, various modifications and changes in the composition and method will occur to those skilled in the art. All such modifications coming within the scope of the appended claims are intended to be included therein.

## Claims

1. Use of a composition comprising a biocompatible polymer, an inorganic contrast agent selected from the group consisting of tantalum, tantalum oxide, barium sulfate, gold, tungsten and platinum, and a biocompatible solvent, for the manufacture of a medicament for sterilizing a female mammal, wherein said composition is to be delivered to the fallopian tube of the female mammal under conditions such that a polymer precipitate forms in situ in the fallopian tube thereby sterilizing the female mammal.

2. The use according to claim 1, wherein said biocompatible polymer is selected from the group consisting of cellulose acetate polymers, ethylene vinyl alcohol copolymers, and polyacrylates.

3. The use according to claim 2, wherein said biocompatible polymer is a cellulose acetate polymer or an ethylene vinyl alcohol copolymer.

4. The use according to claim 1, wherein said biocompatible solvent is selected from the group consisting of dimethylsulfoxide, ethanol, and acetone.

5. The use according to claim 4, wherein said biocompatible solvent is dimethylsulfoxide.

6. The use according to claim 1, wherein said composition is to be delivered into the fallopian tube via a catheter.

7. The use according to claim 1, wherein said composition is to be delivered into the fallopian tube via a needle.

8. Use of a composition comprising a biocompatible polymer, an inorganic contrast agent selected from the group consisting of tantalum, tantalum oxide, barium sulfate, gold, tungsten and platinum, and a first biocompatible solvent, for the manufacture of a medicament for reversibly sterilizing a female mammal, wherein said composition is to be delivered to the fallopian tube of the female mammal under conditions such that a polymer precipitate forms in situ in the fallopian tube to thereby sterilize the female mammal, and a second biocompatible solvent is to be contacted with said polymer precipitate formed in the fallopian tube under conditions such that said polymer dissolves in said second biocompatible solvent to thereby reverse said sterilization of the female mammal.

9. The use according to claim 8, wherein said biocompatible polymer is selected from the group consisting of cellulose acetate polymers, ethylene vinyl alcohol copolymers and polyacrylates.

10. The use according to claim 9, wherein said biocompatible polymer is a cellulose acetate polymer or an ethylene vinyl alcohol copolymer.

11. The use according to claim 8, wherein said first biocompatible solvent and said second biocompatible solvent are the same.

12. The use according to claim 8, wherein each of said first biocompatible solvent and said second biocompatible solvent is selected from the group consisting of dimethylsulfoxide, ethanol and acetone.

13. The use according to claim 12, wherein said first biocompatible solvent and said second biocompatible solvent are dimethylsulfoxide.

14. The use according to claim 8, wherein said composition is to be delivered into the fallopian tube via a catheter or a needle.

15. The use according to claim 8, wherein said second biocompatible solvent is to be delivered into the fallopian tube via a catheter or a needle.

16. The use according to claim 8, wherein said second biocompatible solvent further comprises a contrast agent.

## Patentansprüche

1. Verwendung einer Zusammensetzung, die ein biokompatibles Polymer, ein anorganisches Kontrastmittel, ausgewählt aus der Gruppe, die besteht aus Tantal, Tantaloxid, Bariumsulfat, Gold, Wolfram und Platin, und ein biokompatibles Lösungsmittel umfasst, zur Herstellung eines Arzneimittels zum Sterilisieren eines weiblichen Säugetiers, wobei die genannte Zusammensetzung in den Eileiter des weiblichen Säugetiers unter solchen Bedingungen eingeführt werden soll, dass in dem Eileiter in situ ein Polymer präzipitiert wird, wodurch das weibliche Säugetier sterilisiert wird.

2. Verwendung nach Anspruch 1, bei der das biokompatible Polymer ausgewählt wird aus der Gruppe, die besteht aus Celluloseacetat-Polymeren, Ethylen/Vinylalkohol-Copolymeren und Polyacrylaten.

3. Verwendung nach Anspruch 2, bei der das biokompatible Polymer ein Celluloseacetat-Polymer oder ein Ehylen/Vinylalkohol-Copolymer ist.

4. Verwendung nach Anspruch 1, bei der das biokompatible Lösungsmittel ausgewählt wird aus der Gruppe, die besteht aus Dimethylsulfoxid, Ethanol und Aceton.

5. Verwendung nach Anspruch 4, bei der das biokompatible Lösungsmittel Dimethylsulfoxid ist.

6. Verwendung nach Anspruch 1, bei der die Zusammensetzung mittels eines Katheters in den Eileiter eingeführt werden soll.

7. Verwendung nach Anspruch 1, bei der die Zusammensetzung mittels einer Nadel in den Eileiter eingeführt werden soll.

8. Verwendung einer Zusammensetzung, die ein biokompatibles Polymer, ein anorganisches Kontrastmittel, ausgewählt aus der Gruppe, die besteht aus Tantal, Tantaloxid, Bariumsulfat, Gold, Wolfram und Platin, und ein erstes biokompatibles Lösungsmittel umfasst, zur Herstellung eines Arzneimittels für die reversible Sterilisierung eines weiblichen Säugetiers, bei der die Zusammensetzung unter solchen Bedingungen in den Eileiter des weiblichen Säugetiers eingeführt werden soll, dass sich in dem Eileiter in situ ein Polymer-Präzipitat bildet, wodurch das weibliche Säugetier sterilisiert werden soll, und ein zweites biokompatibles Lösungsmittel unter solchen Bedingungen mit dem in dem Eileiter gebildeten Polymer-Präzipitat in Kontakt gebracht werden soll, dass sich das Polymer in dem zweiten biokompatiblen Lösungsmittel löst, wodurch die Sterilisation des weiblichen Säugetiers aufgehoben wird.

9. Verwendung nach Anspruch 8, bei der das biokompatible Polymer ausgewählt wird aus der Gruppe, die besteht aus Celluloseacetat-Polymeren, Ethylen/Vinylalkohol-Copolymeren und Polyacrylaten.

10. Verwendung nach Anspruch 8, bei der das biokompatible Polymer ein Celluloseacetat-Polymer oder ein Ethylen-Vinylalkohol-Copolymer ist.

11. Verwendung nach Anspruch 8, bei der das erste biokompatible Lösungsmittel und das zweite biokompatible Lösungsmittel gleich sind.

12. Verwendung nach Anspruch 8, bei der das erste biokompatible Lösungsmittel und das zweite biokompatible Lösungsmittel jeweils ausgewählt werden aus der Gruppe, die besteht aus Dimethylsulfoxid, Ethanol und Aceton.

13. Verwendung nach Anspruch 12, bei der das erste biokompatible Lösungsmittel und das zweite biokompatible Lösungsmittel Dimethylsulfoxid sind.

14. Verwendung nach Anspruch 8, bei der die Zusammensetzung mittels eines Katheters oder einer Nadel in den Eileiter eingeführt werden soll.

15. Verwendung nach Anspruch 8, bei der das zweite biokompatible Lösungsmittel mittels eines Katheters oder einer Nadel in den Eileiter eingeführt werden soll.

16. Verwendung nach Anspruch 8, bei der das zweite biokompatible Lösungsmittel außerdem ein Kontrastmittel umfasst.

## Revendications

1. Utilisation d'une composition comprenant un polymère biocompatible, un agent de contraste minéral choisi dans le groupe comprenant le tantale, l'oxyde de tantale, le sulfate de baryum, l'or, le tungstène et le platine, et un solvant biocompatible, pour fabriquer un médicament destiné à la stérilisation d'un mammifère femelle, dans laquelle ladite composition doit être administrée dans la trompe de Fallope du mammifère femelle dans des conditions telles qu'un précipité polymère se forme *in situ* dans la trompe de Fallope, stérilisant ainsi le mammifère femelle.

2. Utilisation selon la revendication 1, dans laquelle ledit polymère biocompatible est choisi dans le groupe comprenant les polymères d'acétate de cellulose, les copolymères d'éthylène/alcool vinylique et les polyacrylates.

3. Utilisation selon la revendication 2, dans laquelle ledit polymère biocompatible est un polymère d'acétate de cellulose ou un copolymère d'éthylène/alcool vinylique.

4. Utilisation selon la revendication 1, dans laquelle ledit solvant biocompatible est choisi dans le groupe comprenant le diméthylsulfoxyde, l'éthanol et l'acétone.

5. Utilisation selon la revendication 4, dans laquelle ledit solvant biocompatible est le diméthylsulfoxyde.

6. Utilisation selon la revendication 1, dans laquelle ladite composition doit être administrée dans la trompe de Fallope par l'intermédiaire d'un cathéter.

7. Utilisation selon la revendication 1, dans laquelle ladite composition doit être administrée dans la trompe de Fallope par l'intermédiaire d'une aiguille.

8. Utilisation d'une composition comprenant un polymère biocompatible, un agent de contraste minéral choisi dans le groupe comprenant le tantale, l'oxyde de tantale, le sulfate de baryum, l'or, le tungstène et le platine, et un premier solvant biocompatible, pour fabriquer un médicament destiné à la stérilisation réversible d'un mammifère femelle, dans laquelle ladite composition doit être administrée dans la trompe de Fallope du mammifère femelle dans des conditions telles qu'un précipité polymère se forme *in situ* dans la trompe de Fallope pour ainsi stériliser le mammifère femelle, et un second solvant biocompatible doit être mis en contact avec ledit précipité polymère formé dans la trompe de Fallope dans des conditions telles que ledit polymère se dissout dans ledit second solvant biocompatible pour ainsi renverser ladite stérilisation du mammifère femelle.

9. Utilisation selon la revendication 8, dans laquelle ledit polymère biocompatible est choisi dans le groupe comprenant les polymères d'acétate de cellulose, les copolymères d'éthylène/alcool vinylique et les polyacrylates.

10. Utilisation selon la revendication 9, dans laquelle ledit polymère biocompatible est un polymère d'acétate de cellulose ou un copolymère d'éthylène/alcool vinylique.

11. Utilisation selon la revendication 8, dans laquelle ledit premier solvant biocompatible et ledit second solvant biocompatible sont identiques.

12. Utilisation selon la revendication 8, dans laquelle chacun desdits premier solvant biocompatible et second solvant biocompatible est choisi dans le groupe comprenant le diméthylsulfoxyde, l'éthanol et l'acétone.

13. Utilisation selon la revendication 12, dans laquelle ledit premier solvant biocompatible et ledit second solvant biocompatible sont le diméthylsulfoxyde.

14. Utilisation selon la revendication 8, dans laquelle ladite composition doit être administrée dans la trompe de Fallope par l'intermédiaire d'un cathéter ou d'une aiguille.

15. Utilisation selon la revendication 8, dans laquelle ledit second solvant biocompatible doit être administré dans la trompe de Fallope par l'intermédiaire d'un cathéter ou d'une aiguille.

16. Utilisation selon la revendication 8, dans laquelle ledit second solvant biocompatible comprend en outre un agent de contraste.
